# EUROPEAN PATENT APPLICATION

(11) **EP 4 495 226 A1**
(43) Date of publication of application: **22.01.2025**
(21) Application number: 23771133.8
(22) Date of filing: 17.03.2023
(51) Int. Cl.: C12N 1/20, C12N 9/88, C12N 15/77, C12P 13/06, C12R 1/15

(54) **MICROORGANISM WITH ENHANCED ACTIVITY OF ASPARTATE 1-DECARBOXYLASE DERIVED FROM TRIBOLIUM CASTANEUM, AND USES THEREOF**

(30) Priority: 18.03.2022 KR 20220034234
(71) Applicant: CJ CheilJedang Corporation, Seoul 04560 (KR)
(72) Inventor: JANG, Yeon-Jae, Seoul 04560 (KR); SHIN, Kwang Soo, Seoul 04560 (KR); SO, Yee-Seul, Seoul 04560 (KR); LEE, Kwang Woo, Seoul 04560 (KR)
(74) Representative: Reddie & Grose LLP
(86) International application number: PCT/KR2023/003573
(87) International publication number: WO 2023/177254

(57) **Abstract**

The present application provides: a microorganism with enhanced activity of aspartate 1-decarboxylase derived from *Tribolium castaneum*; a composition for producing beta-alanine and/or a beta-alanine-derived compound, the composition comprising the microorganism; and a method for producing beta-alanine and/or a beta-alanine-derived compound, the method comprising a step for culturing the microorganism. The productivity of beta-alanine and/or a beta-alanine-derived compound is excellent.

## Description

### [TECHNICAL FIELD]

### Cross-reference to related application(s)

The present application claims the benefit of the priority based on Korean Patent Application No. 10-2022-0034234 filed on March 18, 2022, and the entire contents disclosed in the document of the corresponding Korean patent application are incorporated as a part of the present description.

Provided are a microorganism with enhanced activity of aspartate 1-decarboxylase derived from *Tribolium castaneum,* a composition for producing beta-alanine and/or beta-alanine-derived compounds comprising the microorganism, and a method for producing beta-alanine and/or beta-alanine-derived compounds comprising culturing the microorganism.

### [BACKGROUND ART]

Beta-alanine (β-alanine) is a naturally occurring β-amino acid in which an amino group is combined to the β carbon. Beta-alanine is found naturally in foods such as poultry, meat and fish, and is used to synthesize carnosine in muscles.

Beta-alanine-derived compounds, such as beta-alanine and pantothenic acid, are one of commercially important substances applied in various industrial fields such as health supplementary foods, foods, pharmaceuticals, animal feeds, and the like.

Accordingly, development of a microorganism having an advantageous effect in biotechnologically preparing beta-alanine and/or beta-alanine-derived compounds, and a technology for preparing beta-alanine and/or beta-alanine-derived compounds using the same with high efficiency is required.

### [PRIOR ART]

### [PATENT DOCUMENT]

(Patent document 1) U.S. Patent No. 7718205

### [DISCLOSURE]

### [TECHNICAL PROBLEM]

The present application provides a microorganism with enhanced activity of aspartate 1-decarboxylase, and enhanced production ability of beta-alanine and/or beta-alanine-derived compounds.

The present application provides a composition for producing beta-alanine and/or beta-alanine-derived compounds comprising the microorganism.

The present application provides a method for producing beta-alanine and/or beta-alanine-derived compounds, comprising culturing the microorganism in a medium.

The present application provides a use of the microorganism for using in production of beta-alanine and/or beta-alanine-derived compounds.

The present application provides a use of the microorganism for using in preparation of a composition for producing beta-alanine and/or beta-alanine-derived compounds.

### [TECHNICAL SOLUTION]

The present application relates to a use for production and/or enhancement of production ability of beta-alanine and/or beta-alanine-derived compounds of aspartate 1-decarboxylase, for example, aspartate 1-decarboxylase derived from *Tribolium castaneum.*

One aspect provides a microorganism (or strain, recombinant cell) with enhanced activity of aspartate 1-decarboxylase.

In the present application, aspartate 1-decarboxylase (or PanD protein) may mean a protein having aspartate 1-decarboxylase activity. The aspartate 1-decarboxylase may be derived from *Tribolium castaneum* (Red flour beetle), *E. coli* (*Escherichia coli*), *Bacillus subtilis, Serratia rubidaea, Corynebacterium glutamicum,* or *Pseudomonas* sp. bacteria (*Pseudomonas* sp.), but not limited thereto. In one embodiment, the aspartate 1-decarboxylase may be derived from *Tribolium castaneum,* and the sequence of the aspartate 1-decarboxylase derived from *Tribolium castaneum* may be obtained from a known database, NCBI, and for example, it may be represented by NCBI Reference Sequence: NP_001096055.1. In one embodiment, the aspartate 1-decarboxylase derived from *Tribolium castaneum may* have or comprise the amino acid sequence of SEQ ID NO: 27, or consist of the amino acid sequence, or be essentially consisting of the amino acid sequence. Specifically, the aspartate 1-decarboxylase may consist of a polypeptide described as the amino acid sequence of SEQ ID NO: 27.

In one embodiment, the aspartate 1-decarboxylase protein may have homology or identity of at least 70% or more, 75% or more, 80% or more, 85% or more, 90% or more, 95% or more, 96% or more, 97% or more, 98% or more, 98.2% or more, 98.4% or more, 98.6% or more, 98.8% or more, 98.9% or more, 99% or more, 99.1% or more, 99.3% or more, 99.5% or more, 99.7% or more, or 99.9% or more to the amino acid sequence described as SEQ ID NO: 27, or comprise the sequence, or consist of the sequence, but not limited thereto. In addition, as long as it is an amino acid sequence which has such homology or identity and exhibits efficacy corresponding to aspartate 1-decarboxylase, a variant having an amino acid sequence in which some sequences are deleted, modified, substituted, conservatively substituted or added may be also included in the aspartate 1-decarboxylase. For example, it is a case with addition or deletion of a sequence which does not change the activity of aspartate 1-decarboxylase at the N-terminus, C-terminus, and/or inside of the amino acid sequence, mutation that may occur naturally, silent mutation or conservative substitution.

The "conservative substitution" means substituting one amino acid with another amino acid having similar structural and/or chemical properties. Such amino acid substitution may generally occur based on similarity in polarity, charge, solubility, hydrophobicity, hydrophilicity, and/or amphipathic nature of a residue. Commonly, the conservative substitution may have little effect or have no effect on the activity of the protein or polypeptide.

In the present application, the aspartate 1-decarboxylase may be a polypeptide having activity of aspartate 1-decarboxylase encoded by an aspartate 1-decarboxylase gene. The aspartate 1-decarboxylase gene may be derived from *Tribolium castaneum* (Red flour beetle), *E. coli* (*Escherichia coli*), *Bacillus subtilis*, *Serratia rubidaea, Corynebacterium glutamicum*, or *Pseudomonas* sp. bacteria (*Pseudomonas* sp.), but not limited thereto. In one embodiment, the aspartate 1-decarboxylase gene may be derived from *Tribolium castaneum.* The sequence of the aspartate 1-decarboxylase gene derived from *Tribolium castaneum* may be obtained from a known database, NCBI, and for example, it may be represented by NCBI Reference Sequence: NM_001102585.1. In one embodiment, the aspartate 1-decarboxylase gene derived from *Tribolium castaneum* may have or comprise the nucleic acid sequence of SEQ ID NO: 38, or consist of the nucleic acid sequence, or be essentially consisting of the nucleic acid sequence. Specifically, the aspartate 1-decarboxylase gene may consist of the nucleic acid sequence of SEQ ID NO: 38. In one embodiment, the aspartate 1-decarboxylase gene may comprise a nucleic acid sequence having homology or identity of at least 70%, 75%, 80%, 85%, 90%, 95%, 96%, 97%, 98%, 99%, 99.5%, 99.7% or 99.9% or more to the nucleic acid sequence of SEQ **ID** NO: 38, or consist of the sequence.

In the present application, that a polynucleotide or polypeptide "has or comprises a specific nucleic acid sequence (base sequence) or amino acid sequence or consists of the sequence or is essentially consisting of the sequence" may mean that the polynucleotide or polypeptide may mean essentially comprise the specific nucleic acid sequence (base sequence) or amino acid sequence, and it may be interpreted to comprise a "substantially equivalent sequence" in which a mutation (deletion, substitution, modification, and/or addition) is applied to the specific nucleic acid sequence (base sequence) or amino acid sequence within the range of maintaining the original functions and/or desired functions of the polynucleotide or polypeptide (or not to exclude the mutation). In one embodiment, a polynucleotide or polypeptide "has or comprise a specific nucleic acid (base sequence) or amino acid sequence, consists of the sequence, or is essentially consisting of the sequence" may mean that the polynucleotide or polypeptide (i) essentially comprises the specific nucleic acid sequence (base sequence) or amino acid sequence, or (ii) consists of a nucleic acid sequence or amino acid sequence having homology or identity of 70% or more, 80% or more, 85% or more, 90% or more, 91% or more, 92% or more, 93% or more, 94% or more, 95% or more, 96% or more, 97% or more, 98% or more, 98% or more, 99% or more, 99.5% or more, or 99.9% or more to the specific nucleic acid sequence (base sequence) or amino acid sequence or essentially comprises the same, and maintain the original functions and/or desired functions. In one embodiment, the desired functions may mean functions to increase (enhance) or give production ability of beta-alanine and/or beta-alanine-derived compounds of a microorganism.

In the present application, the term 'homology' or 'identity' refers to a degree of similarity between two given amino acid sequences or base sequences and may be expressed as a percentage. The terms, homology and identity may be often used interchangeably.

The sequence homology or identity of the conserved polynucleotide or polypeptide may be determined by a standard array algorithm, and a default gap penalty established by a used program may be used together. Substantially, a homologous or identical sequence may be generally hybridized to the entire or a part of a sequence under a medium or high stringent condition. It is obvious that the hybridization also includes hybridization with a polynucleotide containing a common codon or codon considering codon degeneracy in the polynucleotide.

Whether any two polynucleotide or polypeptide sequences have homology, similarity or identity, may be determined, for example, using a default parameter such as Pearson et al (1988) [Proc. Natl. Acad. Sci. USA 85]: 2444 using a known computer algorithm such as "FASTA" program. Otherwise, it may be determined using Needleman-Wunsch algorithm (Needleman and Wunsch, 1970, J. Mol. Biol. 48: 443-453), as performed in Needleman program of EMBOSS package (EMBOSS: The European Molecular Biology Open Software Suite, Rice et al., 2000, Trends Genet. 16: 276-277) (version 5.0.0 or later version) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, using BLAST, or ClustalW of National Center for Biotechnology Information Database, the homology, similarity or identity may be determined.

The homology, similarity, or identity of the polynucleotide or polypeptide may be determined by compared sequence information using for example, GAP computer program such as Needleman et al. (1970), J Mol Biol. 48:443, for example, known in Smith and Waterman, Adv. Appl. Math (1981) 2:482. In summary, the GAP program may be defined as a value of dividing a total number of symbols in the shorter of two sequences by the number of similar aligned symbols (i.e., nucleotides or amino acids). The default parameters for the GAP program may comprise (1) a binary comparison matrix (containing values of 1 for identity and 0 for non-identity) and a weighted comparison matrix of Gribskov et al (1986) Nucl. Acids Res. 14: 6745, disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a 3.0 penalty for each gap and an additional 0.10 penalty for each symbol in each gap (or gap opening penalty 10, gap extension penalty 0.5); and (3) no penalty for end gaps.

In the present application, the term, "microorganism (or, strain)" includes all of wild-type microorganisms or microorganisms in which genetic modification occurs naturally or artificially, and may be a microorganism comprising genetic modification for production of a targeted polypeptide, protein or product (for example, aspartate 1-decarboxylase), which is a microorganism in which specific mechanism is enhanced or weakened due to causes such as that a foreign gene is inserted or activity of an intrinsic gene is enhanced or weakened.

In the present application, the term, "enhancement" of the polypeptide activity, means that the activity of the polypeptide is increased compared to the intrinsic activity. The enhancement may be interchangeably used with terms such as activation, up-regulation, overexpression, increase and the like. Herein, the activation, up-regulation, overexpression, and increase may include showing activity which is not originally present, or showing activity improved compared to the intrinsic activity or activity before modification. The "intrinsic activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide "is enhanced", "is up-regulated", "is overexpressed" or "increases" compared to the intrinsic activity, refers to that it is improved compared to the activity and/or concentration (expression level) of a specific polypeptide originally possessed by a parent strain before trait changes or non-modified microorganism.

The enhancement may be achieved by introducing a foreign polypeptide, or enhancement of activity of an intrinsic polypeptide and/or concentration (expression level). Whether the activity of the polypeptide is enhanced may be confirmed by the degree of activity, expression level of the corresponding polypeptide, or an increase of the amount of products released from the corresponding polypeptide.

For the enhancement of the activity of the polypeptide, various methods well known in the art may be applied, and there is no limitation, as long as the activity of a target polypeptide can be enhanced compared to a microorganism before modification. Specifically, genetic engineering and/or protein engineering well known to those skilled in the art, which are routine methods of molecular biology may be used, but not limited thereto (For example, Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present application may be
1) an increase of a copy number in cells of a polynucleotide encoding a polypeptide;
2) replacement of a gene expression regulatory region on chromosome encoding a polypeptide with a sequence having strong activity;
3) modification of a base sequence encoding an initiation codon or 5'-UTR area of a gene transcriptome encoding a polypeptide;
4) modification of an amino acid sequence of the polypeptide so that the activity of the polypeptide is enhanced;
5) modification of a polynucleotide sequence encoding the polypeptide so that the activity of the polypeptide is enhanced (for example, modification of a polynucleotide of the polypeptide gene so as to encode a polypeptide modified to enhance the activity of the polypeptide);
6) introduction of a foreign polypeptide showing activity of a polypeptide or a foreign polynucleotide encoding the same;
7) codon optimization of a polynucleotide encoding a polypeptide;
8) modification or chemical modification by analyzing the tertiary structure of a polypeptide and select an exposed site; or
9) a combination of at least 2 selected from the 1) to 8), but not particularly limited thereto.

More specifically, the 1) increase of a copy number in cells of a polynucleotide encoding a polypeptide may be achieved by introduction of a vector which can replicate and function regardless of a host, in which a polynucleotide encoding the corresponding polypeptide is operably linked. Otherwise, it may be achieved by introduction of 1 copy or 2 copies or more of the polynucleotide encoding the corresponding polypeptide into chromosome in a host cell. The introduction into the chromosome may be performed by introducing a vector capable of inserting the polynucleotide into chromosome of the host cell into the host cell, but not limited thereto. The vector is as described above.

The 2) replacement of a gene expression regulatory region (or expression regulatory sequence) on chromosome encoding a polypeptide with a sequence having strong activity, may be for example, occurrence of mutation in the sequence by deletion, insertion, non-conservative or conservative substitution or a combination thereof so as to further intensify activity of the expression regulatory region, or replacement with a sequence having stronger activity. The expression regulatory region is not particularly limited thereto, but may comprise a promoter, an operator sequence, a sequence encoding a ribosome binding site, and a sequence regulating termination of transcription and translation and the like. As one example, the original promoter may be replaced with a strong promoter, but not limited thereto.

Examples of the known strong promoter include CJ1 to CJ7 promoters (U.S. Patent US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent US 10584338 B2), O2 promoter (U.S. Patent US 10273491 B2), tkt promoter, yccA promoter, and the like, but not limited thereto.

The 3) modification of a base sequence encoding an initiation codon or 5'-UTR area of a gene transcriptome encoding a polypeptide may be for example, substituting it with a base sequence encoding another initiation codon with a higher polypeptide expression rate compared to the intrinsic initiation codon, but not limited thereto.

The modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have stronger activity for the amino acid sequence of the polypeptide or a polynucleotide sequence encoding the polypeptide so as to strengthen the activity of the polypeptide, but not limited thereto. The replacement may be performed specifically by inserting a polynucleotide into chromosome by homologous recombination, but not limited thereto. The vector used then may further comprise a selection marker for confirming chromosome insertion. The selection marker is as described above.

The 6) introduction of a foreign polypeptide showing activity of a polypeptide may be introduction of a foreign polynucleotide encoding a polypeptide showing the same/similar activity to the polypeptide into a host cell. The foreign polynucleotide is not limited in its origin or sequence as long as it shows the same/similar activity to the polypeptide. The method used for the introduction may be performed by those skilled in the art by appropriately selecting a known transformation method, and by expressing the introduced polynucleotide in a host cell, a polypeptide may be produced and its activity may be increased.

The 7) codon optimization of a polynucleotide encoding a polypeptide may be codon optimization so that transcription or translation of an intrinsic polynucleotide is increased in a host cell, or codon optimization so that a foreign polynucleotide is optimized in a host cell to achieve transcription and translation.

The 8) modification or chemical modification by analyzing the tertiary structure of a polypeptide and select an exposed site may determine template protein candidates depending on the degree of similarity of sequences by comparing sequence information of a polypeptide to be analyzed, and confirm the structure based on this, and select an exposed site to be modified or chemically modified, and transform or modify it.

Such enhancement of the polypeptide activity, may be that the activity or concentration expression level of the corresponding polypeptide is increased based on the activity or concentration of the polypeptide expressed in a microbial strain which is a wild-type or before modification, or that the amount of products produced from the corresponding polypeptide is increased, but not limited thereto.

In the present application, the term, "weakening" of the polypeptide, is a concept including both reducing activity or having no activity compared to the intrinsic activity. The weakening may be interchangeably used with terms such as inactivation, deficiency, down-regulation, decrease, reduce, attenuation, and the like. The weakening may also include cases where the activity of the polypeptide itself is reduced or removed compared to activity of the polypeptide possessed by the original microorganism due to mutation of a polynucleotide encoding the polypeptide (or protein, for example, aspartate 1-decarboxylase) and the like, cases where the overall polypeptide activity level and/or concentration (expression level) is lower than a natural strain in cells due to inhibition of expression or inhibition of translation into a polypeptide of a gene of a polynucleotide encoding the same, and the like, cases where expression of the polynucleotide is not done at all, and/or cases where there is no activity of the polypeptide even if the polynucleotide is expressed. The "intrinsic activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide is "inactivated, deficient, decreased, down-regulated, reduced, or attenuated" compared to the intrinsic activity, refers to that it is lowered compared to the activity of a specific polypeptide originally possessed by a parent strain before trait changes or non-modified microorganism.

This weakening of the activity of the polypeptide (or protein, for example, aspartate 1-decarboxylase) may be performed by any method known in the art, but not limited thereto, and may be achieved by application of various methods well known in the art (for example, Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide (or protein, for example, aspartate 1-decarboxylase; hereinafter, described as polypeptide) may be
1) deficiency of the entire or a part of a gene encoding a polypeptide;
2) modification of an expression regulatory region (or expression regulatory sequence) so that expression of a gene encoding a polypeptide is reduced;
3) modification (for example, deletion/substitution/addition of at least one amino acid in the amino acid sequence) of an amino acid sequence composing the polypeptide so that the activity of the polypeptide is removed or weakened;
4) modification (for example, addition/substitution/addition of at least one nucleic acid base in the nucleic acid base sequence of the polypeptide gene to encode a polypeptide modified so that the activity of the polypeptide is removed or weakened) of a gene sequence encoding the polypeptide so that the activity of the polypeptide is removed or weakened;
5) modification of a base sequence encoding an initiation codon or 5'-UTR area of a gene transcriptome encoding a polypeptide;
6) introduction of an antisense oligonucleotide (for example, antisense RNA) complementarily binding to transcriptome of a gene encoding a polypeptide;
7) addition of a sequence complementary to Shine-Dalgarno sequence in front of the Shine-Dalgarno sequence of a gene encoding a polypeptide so as to form a secondary structure incapable of attachment of ribosome;
8) addition of a promoter transcribed in the opposite direction at the 3' end of an ORF (open reading frame) of a gene sequence encoding a polypeptide (Reverse transcription engineering, RTE); or
9) a combination of at least 2 selected from the 1) to 8), but not particularly limited thereto.

For example,
the 1) deficiency of the entire or a part of a gene encoding a polypeptide may be removal of the entire polynucleotide encoding an intrinsic target polypeptide in chromosome, replacement with a polynucleotide in which some nucleotides are deleted, or replacement with a marker gene.

In addition, the 2) modification of an expression regulatory region (or expression regulatory sequence) may be mutation occurrence on the expression regulatory region (or expression regulatory sequence) by deletion, insertion, non-conservative or conservative substitution or a combination thereof, or replacement with a sequence having weaker activity. The expression regulatory region includes a promoter, an operator sequence, a sequence encoding a ribosome binding site, and a sequence regulating termination of transcription and translation, but not limited thereto.

Furthermore, The 3) modification of a base sequence encoding an initiation codon or 5'-UTR area of a gene transcriptome encoding a polypeptide may be for example, substituting it with a base sequence encoding another initiation codon with a lower polypeptide expression rate compared to the intrinsic initiation codon, but not limited thereto.

Moreover, the modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have weaker activity or an amino acid sequence or polynucleotide sequence improved to have no activity, but not limited thereto. For example, by forming a stop codon by introducing mutation in a polynucleotide sequence, expression of a gene may be inhibited or weakened, but not limited thereto. The "stop codon" is a codon acting as a signal that does not designate amino acids in the codon on mRNA and notifies that the protein synthesis process is ended, and generally, three kinds of UAA, UAG and UGA may be used as the stop codon.

The 6) introduction of an antisense oligonucleotide (for example, antisense RNA) complementarily binding to transcriptome of a gene encoding a polypeptide may refer to for example, the document [Weintraub, H. et al., Antisense-RNAas a molecular tool for genetic analysis, Reviews - Trends in Genetics, Vol. 1(1) 1986].

The 7) addition of a sequence complementary to Shine-Dalgarno sequence in front of the Shine-Dalgarno sequence of a gene encoding a polypeptide so as to form a secondary structure incapable of attachment of ribosome may make mRNA translation impossible or inhibit its speed.

The 8) addition of a promoter transcribed in the opposite direction at the 3' end of an ORF (open reading frame) of a gene sequence encoding a polypeptide (Reverse transcription engineering, RTE) may create an antisense nucleotide complementary to the transcriptome of the gene encoding the polypeptide to weaken the activity.

The microorganism of the present application may be a microorganism (for example, recombinant microorganism) genetically modified through a vector so that the activity of aspartate 1-decarboxylase or a polynucleotide encoding the same is enhanced, but not limited thereto. The vector is as described above.

The microorganism (or strain, recombinant cell) of the present application may be a microorganism which has production ability of beta-alanine and/or beta-alanine-derived compounds, or has enhanced production ability (or production) of beta-alanine and/or beta-alanine-derived compounds.

The microorganism of the present application may be a microorganism in which production ability of beta-alanine and/or beta-alanine-derived compounds is given or enhanced, as the activity of aspartate 1-decarboxylase is introduced or enhanced into a microorganism naturally having no production ability of beta-alanine and/or beta-alanine-derived compounds, or a microorganism having production ability of beta-alanine and/or beta-alanine-derived compounds, but not limited thereto. The microorganism in which the production ability of beta-alanine and/or beta-alanine-derived compounds is given or enhanced may be a microorganism in which aspartate 1-decarboxylase derived from *Tribolium castaneum* is introduced.

That the microorganism (or strain, recombinant cell) has enhanced production ability (or production) of beta-alanine or beta-alanine-derived compounds or has production ability of beta-alanine or beta-alanine-derived compounds may mean that the microorganism (or strain, recombinant cell) has enhanced production ability of beta-alanine or beta-alanine-derived compounds compared to a non-modified microorganism, a cell before recombination, a parent strain, a wild-type strain, and/or a microorganism in which aspartate 1-decarboxylase derived from other microorganism is introduced, or that the production ability of beta-alanine or beta-alanine-derived compounds is given, differently from a non-modified microorganism, a cell before recombination, a parent strain, and/or a wild-type strain which have no production ability of beta-alanine or beta-alanine-derived compounds.

The microorganism with enhanced activity of aspartate 1-decarboxylase according to one embodiment may have improved (increased) production ability of beta-alanine and/or beta-alanine-derived compounds, compared to a microorganism before enhanced, that is, the same kind of non-modified microorganism. In the present application, "non-modified microorganism" does not exclude a strain comprising mutation that can occur naturally in a microorganism, and may mean a wild-type strain or natural strain itself, or a strain before traits are changed due to genetic mutation caused by natural or artificial factors. For example, the non-modified microorganism may mean a strain in which the activity of aspartate 1-decarboxylase is not enhanced or is before it is enhanced according to one embodiment (a strain in which a mutation that induces enhancement of activity of aspartate 1-decarboxylase is not introduced or before it is introduced). The "non-modified microorganism" may be interchangeably used with "strain before modification", "microorganism before modification", "non-mutated strain", "non-modified strain", "non-mutated microorganism" or "reference microorganism". That the activity of aspartate 1-decarboxylase is enhanced is as described above. In one embodiment, the non-modified microorganism, which is a subject strain for comparing whether the production ability of beta-alanine and/or beta-alanine-derived compounds is increased, may be wild-type *Corynebacterium glutamicum* ATCC13032 strain.

The microorganism with enhanced activity of aspartate 1-decarboxylase according to one embodiment may be a microorganism in which aspartate 1-decarboxylase derived from *Tribolium castaneum* is introduced, and the microorganism may have enhanced (increased) production ability of beta-alanine and/or beta-alanine-derived compounds, compared to a microorganism in which aspartate 1-decarboxylase derived from another microorganism is introduced. The aspartate 1-decarboxylase derived from another microorganism may be derived from *E. coli (Escherichia coli), Bacillus subtilis, Serratia rubidaea, Corynebacterium glutamicum,* or *Pseudomonas* sp. bacteria (*Pseudomonas* sp.).

The microorganism (or strain, recombinant cell) may include mutation to increase production of beta-alanine and/or beta-alanine-derived compounds additionally, and any location of the mutation and any kind of genes and/or proteins subject to the mutation may be included without limitation, as long as it increases production of beta-alanine and/or beta-alanine-derived compounds. The recombinant cell may be used without limitation as long as it is a cell capable of transformation.

The beta-alanine-derived compounds may be at least one selected from the group consisting of β-nitropropanoate, β-amino-propionitrile, N-acetyl-β-alanine, L-aspartate, anserine, spermine, carnosine, β-alanyl arginine, quinolinate, pantothenate (or pantothenic acid), malonate semialdehyde, malonate, acetylene-monocarboxylate and the like, but not limited thereto.

As one example, the microorganism (or strain, recombinant cell) with improved (increased) production ability (or production) of the beta-alanine and/or beta-alanine-derived compounds may have production ability of beta-alanine and/or beta-alanine-derived compounds increased by about 10% or more, about 20% or more, about 30% or more, about 50% or more, about 100% or more, about 200% or more, about 300% or more, about 400% or more, about 500% or more, about 600% or more, about 700% or more, about 800% or more, about 900% or more, about 1,000% or more, about 1,500% or more, about 2,000% or more, about 2,500% or more, or about 3,000% or more, compared to a parent strain before mutated, a non-modified microorganism, or a microorganism in which aspartate 1-decarboxylase derived from another microorganism is introduced, and in one embodiment, it may be increased by about 33.3% or more, about 100% or more, about 175% or more, about 243.75% or more, about 266.6% or more, about 300% or more, about 450% or more, about 500% or more, about 587.5% or more, about 700% or more, or about 2,650% or more, but not limited thereto.

In other embodiment, the microorganism (or strain, recombinant cell) with enhanced production ability (or production) may have production ability (or production) of beta-alanine and/or beta-alanine-derived compounds of about 1.1 times or more, about 1.3 times or more, about 1.5 times or more, about 2 times or more, about 3 times or more, about 4 times or more, about 5 times or more, about 6 times or more, about 7 times or more, about 8 times or more, about 9 times or more, about 10 times or more, about 15 times or more, about 20 times or more, about 25 times or more, or about 30 times or more (the upper limit is not particularly limited, and for example, it may be about 1,000 times or less), compared to a parent strain before mutated, a non-modified microorganism, or a microorganism in which aspartate 1-decarboxylase derived from another microorganism is introduced, and in one embodiment, it may be increased by about 1.3 times or more, about 2 times or more, about 2.75 times or more, about 3.43 times or more, about 3.6 times or more, about 4 times or more, about 5.5 times or more, about 6 times or more, about 6.8 times or more, about 8 times or more, or about 27.5 times or more, but not limited thereto.

In other embodiment, the microorganism (or strain, recombinant cell) with enhanced production ability (or production) may have production ability (or production) of beta-alanine and/or beta-alanine-derived compounds of about 0.5g/L or more, about 1g/L or more, about 1.5g/L or more, about 2.0g/L or more, about 2.5g/L or more, about 3g/L or more, about 3.5g/L or more, about 4g/L or more, about 4.5g/L or more, about 5g/L or more, about 5.5g/L or more, about 6g/L or more, about 7g/L or more, about 8g/L or more, about 9g/L or more, about 10g/L or more, about 15g/L or more, about 20g/L or more, about 25g/L or more, about 30g/L or more (the upper limit is not particularly limited, and for example, it may be about 100g/L or less), compared to a parent strain before mutated, a non-modified microorganism, or a microorganism in which aspartate 1-decarboxylase derived from another microorganism is introduced, and in one embodiment, it may be increased by about 0.6g/L or more, about 1.2g/L or more, about 1.8g/L or more, about 2g/L or more, about 2.4g/L or more, about 3.5g/L or more, about 3.9g/L or more, about 4g/L or more, about 4.5g/L or more, about 4.7g/L or more, about 5.3g/L or more, but not limited thereto.

More specifically, the microorganism (or strain, recombinant cell) with enhanced production ability (or production) may have production ability (or production) of beta-alanine and/or beta-alanine-derived compounds increased by about 33.3%, about 100%, about 175%, about 243.75%, about 266.6%, about 300%, about 450%, about 500%, about 587.5%, about 700%, or about 2,650% (or about 1.3 times, about 2 times, about 2.75 times, about 3.43 times, about 3.6 times, about 4 times, about 5.5 times, about 6 times, about 6.8 times, about 8 times, or about 27.5 times; or about 0.6g/L, about 1.2g/L, about 1.8g/L, about 2g/L, about 2.4g/L, about 3.5g/L, about 3.9g/L, about 4g/L, about 4.5g/L, about 4.7g/L, about 5.3g/L), compared to a parent strain before mutated, a non-modified microorganism, or a microorganism in which aspartate 1-decarboxylase derived from another microorganism is introduced, but not limited thereto.

The term, "about" is a range including all of ±0.5, ±0.4, ±0.3, ±0.2, ±0.1 and the like, and includes all numerical values that are in an equivalent or similar range to the numerical value that follows the term about, but not limited thereto.

In one embodiment, the microorganism with the enhance activity of aspartate 1-decarboxylase may be a *Corynebacterium* sp. microorganism. The *Corynebacterium* sp. microorganism may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris*, and/or *Corynebacterium flavescens.*

Other one aspect of the present application provides a method for producing (or method for preparing) beta-alanine and/or beta-alanine-derived compounds, comprising culturing the microorganism of the present application in a medium.

The method for producing beta-alanine and/or beta-alanine-derived compounds of the present application may comprise culturing the microorganism of the present application in a medium. The microorganism, beta-alanine-derived compounds and the like of the present application are as described above.

In the present application, "culturing" means growing a microorganism comprising with enhanced activity of aspartate 1-decarboxylase of the present application under an appropriately adjusted environmental condition. The culturing process of the present application may be conducted according to an appropriate medium and culturing conditions known in the art. This culturing process may be easily adjusted and used by those skilled in the art depending on the selected strain. Specifically, the culturing may be batch, continuous, and/or fed-batch, but not limited thereto.

In the present application, "medium" refers to a material in which nutrients required for culturing the microorganism with enhanced activity of aspartate 1-decarboxylase of the present application are mixed as main components, and supplies nutrients and growth factors and the like including water which is essential for survival and growth. Specifically, the medium used for culturing the microorganism of the present application and other culturing conditions may be used as long as it is a medium used for culturing a common microorganism without particular limitation, but the microorganism of the present application may be cultured in a common medium containing a suitable carbon source, a nitrogen source, a phosphorus source, an inorganic compound, an amino acid and/or a vitamin and the like by adjusting temperature, pH and the like under an aerobic condition.

Specifically, the culture medium for the microorganism of the present application, for example, the *Corynebacterium* sp. strain may be found in the document ["Manual of Methods for General Bacteriology" by the American Society for Bacteriology (Washington D.C., USA, 1981)].

In the present application, as the carbon source, carbohydrates such as glucose, saccharose, lactose, fructose, sucrose, maltose, etc.; sugar alcohols such as mannitol, sorbitol, etc., organic acids such as pyruvate, lactate, citrate, etc.; amino acids such as glutamic acid, methionine, lysine, etc. and the like, may be included. In addition, natural organic nutrients such as starch hydrates, molasses, blackstrap molasses, rice bran, cassava, sugar cane residues, and corn steep liquor may be used, and specifically, carbohydrates such as glucose and sterilized pre-treated molasses (that is, molasses converted with reducing sugars) and the like may be used, and other carbon sources in an appropriate amount may be variously used without limitation. These carbon sources may be used alone or at least two kinds may be used in combination, but not limited thereto.

As the nitrogen source, inorganic nitrogen sources such as ammonia, ammonium sulfate, ammonium chloride, ammonium citrate, ammonium phosphate, ammonium carbonate, ammonium nitrate and the like; and organic nitrogen sources such as amino acids including glutamic acid, methionine, glutamine and the like, peptone, NZ-amine, meat extract, yeast extract, malt extract, corn steep liquor, casein hydrates, fish or degraded products thereof, defatted soybean cake or degraded products thereof, and the like may be used. These nitrogen sources may be used alone or at least two may be used in combination, but not limited thereto.

As the phosphorus source, potassium phosphate monobasic, potassium phosphate dibasic, or sodium-containing salts corresponding thereto, or the like may be included. As the inorganic compound, sodium chloride, calcium chloride, iron chloride, magnesium sulfate, iron sulfate, manganese sulfate, calcium carbonate and the like may be used, and in addition thereto, amino acids, vitamins, and/or appropriate precursors and the like may be included. These components or precursors may be added in a batch or continuous method. However, it is not limited thereto.

In addition, by adding a compound such as ammonium hydroxide, potassium hydroxide, ammonia, phosphoric acid, sulfuric acid and the like to a medium during culturing of the microorganism of the present application by an appropriate method, the pH of the medium may be adjusted. Furthermore, during culturing, foam generation may be inhibited using an antifoaming agent such as fatty acid polyglycol ester. Moreover, in order to maintain the aerobic state of the medium, oxygen or oxygen-containing gas may be injected into the medium, or in order to maintain the anaerobic and microaerobic state, gas may be not injected or nitrogen, hydrogen or carbon dioxide gas may be injected, but not limited thereto.

The culture temperature in the culturing of the present application may be maintained at 20 to 45°C, specifically, 25 to 40°C, and culturing may be performed for about 10 to 160 hours, but not limited thereto.

The beta-alanine and/or beta-alanine-derived compounds produced by the culturing of the present application may be released to a medium or remain in cells.

The method for producing beta-alanine and/or beta-alanine-derived compounds of the present application may further comprise preparing the microorganism (strain) of the present application, preparing a medium for culturing the microorganism, or a combination thereof (in any order), for example, before the culturing.

The method for producing beta-alanine and/or beta-alanine-derived compounds of the present application may further comprise recovering beta-alanine and/or beta-alanine-derived compounds from a medium (medium in which culture is performed) or microorganism (for example, *Corynebacterium* sp. Strain) according the culturing. The recovering may be further comprised after the culturing.

The recovery may collect targeted beta-alanine and/or beta-alanine-derived compounds using an appropriate method known in the art according to a culture method of the microorganism of the present application, for example, a batch, continuous or fed-batch culture method, or the like. For example, centrifugation, filtration, treatment by a crystallized protein precipitator (salting out), extraction, ultrasonic crushing, ultrafiltration, dialysis, various kinds of chromatography such as molecular sieve chromatography (gel filtration), adsorption chromatography, ion exchange chromatography, affinity chromatography, and the like, HPLC or a combination of these methods may be used, and using an appropriate method known in the art, targeted beta-alanine and/or beta-alanine-derived compounds may be recovered from a medium or microorganism.

In addition, the method for producing beta-alanine and/or beta-alanine-derived compounds of the present application may additionally comprise a purification step. The purification may be performed, using an appropriate method known in the art. In one embodiment, when the method for producing beta-alanine and/or beta-alanine-derived compounds of the present application comprises both the recovery step and purification step, the recovery step and purification step may be continuously or non-continuously performed regardless of the order, or may be performed by being integrated as one step, but not limited thereto

Other one aspect of the present application provides a composition for producing beta-alanine and/or beta-alanine-derived compounds comprising the microorganism of the present application, a medium in which the microorganism is cultured, or a combination thereof.

The composition of the present application may further comprise any suitable excipient commonly used in a composition for producing beta-alanine and/or beta-alanine-derived compounds, and such an excipient may be for example, a preservative, wetting agent, dispersant, suspending agent, buffer, stabilizer, or tonicifying agent, or the like, but not limited thereto.

In the composition of the present application, the microorganism (strain), medium, beta-alanine-derived compounds and the like are as described in other aspects above.

Other aspect of the present application provides a use of the microorganism of the present application for using in production of beta-alanine and/or beta-alanine-derived compounds or preparation of a composition for producing beta-alanine and/or beta-alanine-derived compounds. The microorganism of the present application, beta-alanine and/or beta-alanine-derived compounds, or composition for producing beta-alanine and/or beta-alanine-derived compounds are as described above.

### [ADVANTAGEOUS EFFECTS]

The present application provides a microorganism with enhanced activity of aspartate 1-decarboxylase derived from *Tribolium castaneum,* and the microorganism has excellent production ability of beta-alanine and/or beta-alanine-derived compounds.

### [MODE FOR INVENTION]

Hereinafter, the present invention will be described in more detail by the following examples. However, they are intended to illustrate the present invention only, but the scope of the present invention is not limited by these examples.

### Example 1. Investigation and selection of aspartate 1-decarboxylase gene

Aspartate 1-decarboxylase (or PanD) derived from microorganisms are classified into two kinds, in other words, a form having activity upon expression and a form activated by a PanD regulatory factor (PanM, PanD regulatory factor). Therefore, gene investigation of microorganisms having aspartate 1-decarboxylase derived from a microorganism requiring the PanM regulatory factor, and active aspartate 1-decarboxylase was performed. On the basis of prokaryotic cells and eukaryotic cells, based on organisms having aspartate 1-decarboxylase, candidate genes and microorganisms possessing thereof were selected. Among them, aspartate 1-decarboxylase derived from microorganisms with a biosafety level of 1 was selected as Table 1.

**[Table 1]**

| Microorganisms estimated to possess activity of aspartate 1-decarboxylase | | | |
|---|---|---|---|
| Strain | Strain number | Primer sequence number | Plasmid |
| *E. coli* (*Escherichia coli*) | KCTC1116 | SEQ ID NOs: 1, 2 SEQ ID NOs: 3, 4 | pECCG117-panD_panM(EC) |
| *Bacillus subtilis* | ATCC33234 | SEQ ID NOs: 5, 6 | pECCG117-panD(BS) |
| *Serratia rubidaea* | KCTC2927 | SEQ ID NOs: 7, 8 SEQ ID NOs: 9, 10 | pECCG117-panD_panM(SR) |
| *Corynebacterium glutamicum* | ATCC13032 | SEQ ID NOs: 11, 12 | pECCG117-panD(CG) |
| *Pseudomonas* sp. bacteria (*Pseudomonas* sp.) | KCTC72094 | SEQ ID NOs: 13, 14 | pECCG117-panD(PA) |
| *Tribolium castaneum* (Red flour beetle) | GCA_000002335.3 (Genebank accession No) | SEQ ID NOs: 15, 16 | pECCG117-panD(TC) |

### Example 2. Construction of Corynebacterium sp. microorganism in which aspartate 1-decarboxylase derived from foreign microorganism is introduced

PCR was performed using primer sequences of Table 1 (SEQ ID NOs: 1 and 2, SEQ ID NOs: 5 and 6, SEQ ID NOs: 7 and 8, SEQ ID NOs: 11 and 12, SEQ ID NOs: 13 and 14, SEQ ID NOs: 15 and 16) for DNA fragments encoding aspartate 1-decarboxylase using it as a template after extracting genome of the secured microorganisms. PCR was conducted using PfuUltraTM high-reliability DNA polymerase (Stratagene), and PCR conditions were repeating denaturation 95°C, 30 seconds; annealing 55°C, 30 seconds; and polymerization 72°C, 3 minutes, 30 times. As a result, aspartate 1-decarboxylase derived from each microorganism was obtained. In addition, for DNA fragments encoding a PanD regulatory factor (PanM) using genome of *E*. *coli* and *Serratia rubidaea* microorganisms as a template, PCR was performed using the primer sequences of Table 1 (SEQ ID NOs: 3 and 4, SEQ ID NOs: 9 and 10). PCR was conducted using PfuUltraTM high-reliability DNA polymerase (Stratagene), and PCR conditions were repeating denaturation 95°C, 30 seconds; annealing 55°C, 30 seconds; and polymerization 72°C, 3 minutes, 30 times. As a result, the PanD regulatory factor derived from E. *coli* and *Serratia rubidaea* was obtained. In order to secure a lysC promoter derived from *Corynebacterium glutamicum,* PCR was performed for the promoter using the primers 17 and 18 using genomic DNA of *Corynebacterium glutamicum* as a template as same as the above method to obtain DNA fragments. The pECCG117 (Korean Patent No. 10-0057684) vector treated with restriction enzyme BamHI and then heat-treated at 65°C for 20 minutes and the DNA fragments were cloned using TaKaRa's Infusion Cloning Kit according to the provided manual to obtain plasmids, and for them, the name of the vector and information of the introduced gene were indicated in Table 1.

The 6 kinds of the constructed vectors were transformed into *Corynebacterium glutamicum* ATCC 13032 by electroporation, and thereby, strains expressing foreign aspartate 1-decarboxylase (PanD) were constructed.

### Example 3. Investigation of beta-alanine production ability of Corynebacterium sp. microorganism in which aspartate 1-decarboxylase derived from foreign microorganism is expressed

The strains obtained in Example 2 were inoculated into a 250 ml corner-baffled flask containing a production medium of 25 ml consisting of the following composition with a parent strain, and cultured with shaking at 200 rpm at 33 °C for 48 hours. The obtained cultured solution was centrifuged at 20,000 rcf for 10 minutes, and then the supernatant was diluted by 1/10 with TDW (triple distilled water) and then HPLC analysis was performed, and the concentration of beta-alanine was measured, and the result was shown in Table 2 below.

### <Production medium>

Glucose 10%, yeast extract 0.4%, ammonium sulfate 1.5%, potassium phosphate monobasic 0.1%, magnesium sulfate heptahydrate 0.05%, iron sulfate heptahydrate 10 mg/L, manganese sulfate monohydrate 6.7 mg/L, biotin 50 µg/L, thiamine·HCl 100 µg/L, pH 7.2

**[Table 2]**

| Beta-alanine production ability of microorganism expressing aspartate 1-decarboxylase derived from various microorganisms | |
|---|---|
| | Beta-alanine concentration (g/L) |
| ATCC13032 (wild type) | 0.0 |
| ATCC13032pECCG117-panD_panM(EC) | 0.6 |
| ATCC13032pECCG117-panD(BS) | 1.8 |
| ATCC13032pECCG117-panD_panM(SR) | 0.4 |
| ATCC13032pECCG117-panD(CG) | 1.2 |
| ATCC13032pECCG117-panD(PS) | 0.3 |
| ATCC13032pECCG117-panD(TC) | 2.4 |

As a result, as shown in Table 2, it was confirmed that the parent strain, *Corynebacterium glutamicum* ATCC13032 did not produce beta-alanine, and in the strain expressing aspartate 1-decarboxylase of *Corynebacterium glutamicum,* ATCC13032 pECCG117-panD(CG), beta-alanine of about 1.2 g/L was produced. Among the strains expressing aspartate 1-decarboxylase derived from the foreign microorganisms, the strain expressing aspartate 1-decarboxylase derived from *Tribolium castaneum,* ATCC13032 pECCG117-panD(TC) showed the highest beta-alanine productivity as 2.4 g/L.

The above result means that aspartate 1-decarboxylase derived from *Tribolium castaneum* among the 6 kinds of enzymes selected in the present invention could produce beta-alanine much more efficiently.

In the present example, ATCC13032 pECCG117-panD(TC) strain (named *Corynebacterium glutamicum* CV03-5003) confirmed to have the most excellent production ability of beta-alanine was deposited at Korean Culture Center of Microorganisms located in Hongje-dong, Seodaemun-gu, Seoul, Republic of Korea, on November 23, 2021 and given the accession number of KCCM13076P.

### Example 4. Investigation of productivity of beta-alanine-derived compounds of Corynebacterium sp. microorganism in which aspartate 1-decarboxylase derived from foreign microorganism is expressed

In order to confirm the production ability of beta-alanine-derived compounds (pantothenic acid) of aspartate 1-decarboxylase of *Tribolium castaneum* confirmed in Example 3, a microorganism with enhanced activity of 3-methyl-2-oxobutanoate hydroxymethyltransferase (or PanB protein) was constructed.

At first, a vector for defecting the panB gene present in a parent strain was constructed. PCR was performed using genomic DNA of Corynebacterium glutamicum ATCC1303 as a template using primers of SEQ ID NOs: 28 and 29 and SEQ ID NOs: 30 and 31. PCR was performed under conditions of repeating denaturation 95 °C, 30 seconds; annealing 55 °C, 30 seconds; and polymerization 72 °C, 1 minute, 30 times. As a result, gene fragments of 1000 bp from the panB gene upper part and 1000 bp from the panB gene lower part were obtained, respectively, and each amplification product was purified using QIAGEN's PCR Purification kit, and used as an insert DNA fragment for vector construction.

The pDCM2 (Korean Patent No. 2278000) vector treated with restriction enzyme smal and then heat-treated at 65°C for 20 minutes and the DNA fragments (gene fragments of 1000 bp from the panB gene upper part and 1000 bp from the panB gene lower part) were cloned using TaKaRa's Infusion Cloning Kit according to the provided manual so that the molar concentration (M) was to be 2:1:1, thereby constructing the vector pDCM2_ΔpanB for defecting the panB gene on chromosome.

In order to prepare the panB gene derived from E. *coli,* PCR was performed using the genomic DNA of E. *coli* K12 wild-type strain (KCTC1116) as a template and primers 32 and 33. PCR was performed by repeating denaturation 95°C, 30 seconds; annealing 55°C, 30 seconds; and polymerization 72°C, 1 minute and 30 seconds, 30 times, and as a result, a DNA fragment of 795 bp was obtained. In order to secure a lysC promoter derived from *Corynebacterium glutamicum,* PCR was performed in the same way as the method of the above example using the genomic DNA of *Corynebacterium glutamicum* as a template and primers 34 and 35 to obtain a DNA fragment. The pDCM2_ΔpanB vector treated with the restriction enzyme smal and then heat-treated at 65°C for 20 minutes and the obtained DNA fragments were cloned using TaKaRa's Infusion Cloning Kit according to the provided manual so that the molar concentration (M) was to be 2:1:1, to construct the vector pDCM2_ΔpanB::panB(EC) for introducing the panB gene derived from *E. coli* on the chromosome.

By transforming the constructed vector pDMC2_ΔpanB::panB(EC) into *Corynebacterium glutamicum* ATCC13032 by electroporation, and passing through a secondary cross process, strains (ΔpanB::panB(EC)) in which *E*. *coli*-derived panB gene was introduced on the chromosome were obtained, respectively. Appropriate substitution of the *E*. coli-derived panB was confirmed using the following primer combination using MASA (Mutant Allele Specific Amplification) PCR method (Takeda et al., Hum. Mutation, 2, 112-117 (1993)). In other words, the primer combination matching the *E*. *coli panB* gene (SEQ ID Nos: 36 and 35 and SEQ ID Nos: 37 and 32) was first determined by selecting the amplified strain, and the *panB* gene sequence of the selected strain was secondarily confirmed by analyzing it using the primer combination of SEQ ID NO: 36 and SEQ ID NO: 37.

The plasmids obtained in Example 1 were introduced into the ATCC13032 ΔpanB::panB(EC) strain and then the parent strain and the strains were inoculated into a 250 ml corner-baffled flask containing a production medium of 25 ml consisting of the composition as the example, respectively, and then cultured with shaking at 200 rpm at 33 °C for 48 hours to measure the pantothenic production ability.

**[Table 3]**

| Pantothenic acid production ability of microorganism expressing aspartate 1-decarboxylase derived from various microorganisms | |
|---|---|
| | Pantothenic acid concentration (g/L) |
| ATCC13032 ΔpanB::panB(EC) | 0.2 |
| ATCC13032 ΔpanB::panB(EC)pECCG117-panD_panM(EC) | 1.0 |
| ATCC13032 ΔpanB::panB(EC)pECCG117-panD(BS) | 1.6 |
| ATCC13032 ΔpanB::panB(EC)pECCG117-panD_panM(SR) | 0.8 |
| ATCC13032 ΔpanB::panB(EC)pECCG117-panD(CG) | 2.0 |
| ATCC13032 ΔpanB::panB(EC)pECCG117-panD(PS) | 1.5 |
| ATCC13032 ΔpanB::panB(EC)pECCG117-panD(TC) | 5.5 |

As a result, as shown in Table 3 above, it was confirmed that the parent strain, *Corynebacterium glutamicum* ATCC13032ΔpanB::panB(EC) strain hardly produced pantothenic acid, and in the strain expressing aspartate 1-decarboxylase of *Corynebacterium glutamicum* (ATCC13032 ΔpanB::panB(EC) pECCG117-panD(CG)), pantothenic acid of about 2.0 g/L was produced. Among the strains expressing aspartate 1-decarboxylase derived from the foreign microorganism, the strain expressing aspartate 1-decarboxylase derived from *Tribolium castaneum,* ATCC13032 ΔpanB::panB(EC) pECCG117-panD(TC) showed the highest pantothenic acid productivity as 5.5 g/L.

The above result means that the aspartate 1-decarboxylase derived from *Tribolium castaneum* among the 6 kinds of enzymes selected in the present invention could produce not only beta-alanine but also pantothenic acid much more efficiently.

From the above description, those skilled in the art to which the present invention pertains will understand that the present invention can be implemented in other specific forms without changing its technical spirit or essential features. In this regard, the examples described above should be understood as illustrative and not restrictive in all aspects. The scope of the present invention should be interpreted as all changed or modified forms derived from the meaning and scope of the claims described below and equivalent concepts thereof are included in the scope of the present invention.

### [ACCESSION NUMBER]

Name of Depository Authority: Korean Culture Center of Microorganisms
Accession number: KCCM13076P
Date of deposit: 20211123

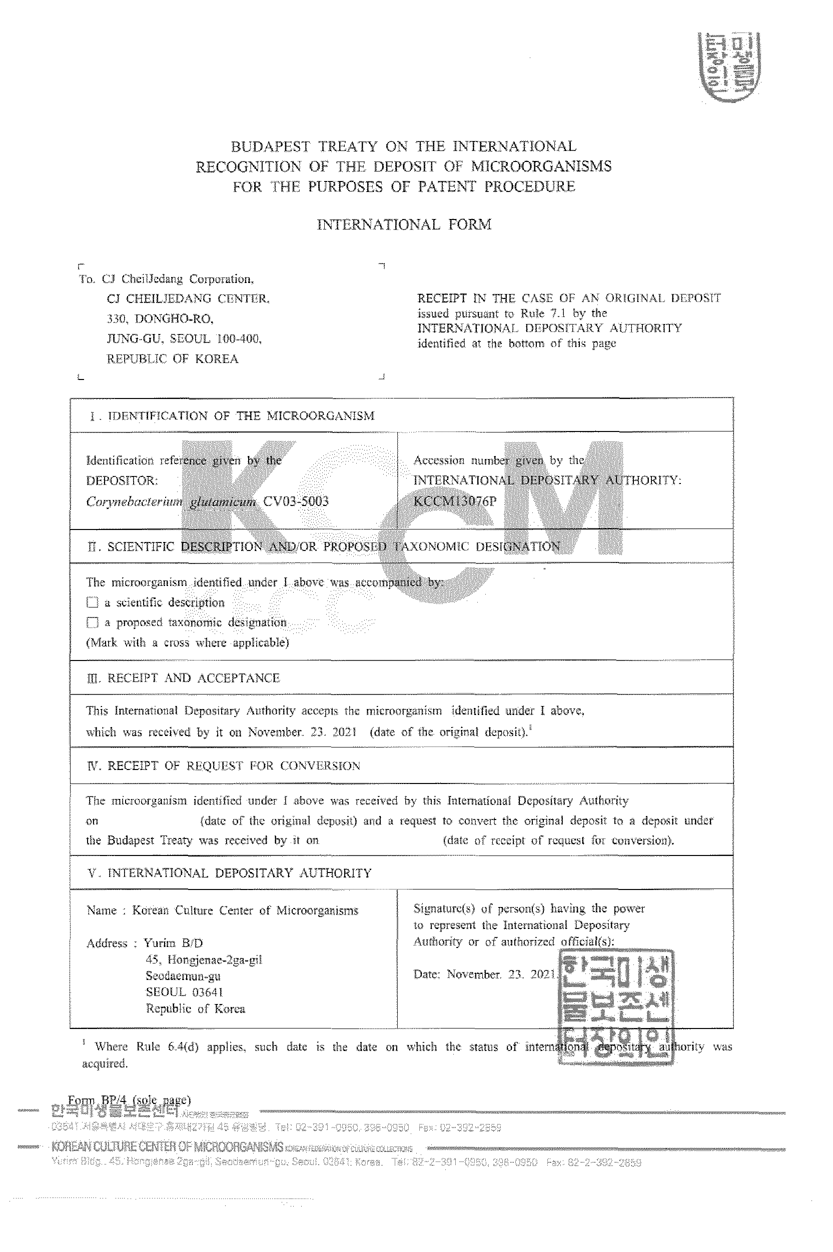

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CV03-5003 | Accession number given by the
INTERNATIONAL DEPOSITARY AUTHORITY:
KCCM13076P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on November. 23. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: November. 23. 2021

¹ Where Rule 6.4(d) applies, such date is the date on which the status of international depositary authority was
acquired.

Form BP/4 (sole page)
한국미생물보존센터 [?]
03641 서울특별시 서대문구 홍제내2가길 45 유림빌딩. Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. A *Corynebacterium* sp. microorganism producing beta-alanine or beta-alanine-derived compound, in which aspartate 1-decarboxylase derived from *Tribolium castaneum* or a polynucleotide encoding the same is introduced.

2. The microorganism according to claim 1, wherein the aspartate 1-decarboxylase is PanD protein.

3. The microorganism according to claim 1, wherein the aspartate 1-decarboxylase comprises an amino acid sequence represented by SEQ ID NO: 27 or an amino acid sequence having sequence identity of at least 95% thereto.

4. The microorganism according to claim 1, wherein the aspartate 1-decarboxylase is encoded by a polynucleotide comprising a nucleic acid sequence of SEQ ID NO: 38.

5. The microorganism according to any one of claims 1 to 4, wherein the *Corynebacterium* sp. microorganism is *Corynebacterium glutamicum.*

6. The microorganism according to any one of claims 1 to 4, wherein the microorganism has increased production ability of beta-alanine or beta-alanine-derived compound, compared to a parent strain or wild type into which the aspartate 1-decarboxylase or a polynucleotide encoding the same is not introduced.

7. A method for producing beta-alanine or beta-alanine-derived compound, comprising culturing a *Corynebacterium* sp. microorganism, into which aspartate 1-decarboxylase derived from *Tribolium castaneum* or a polynucleotide encoding the same is introduced, in a medium.

8. The method according to claim 7, further comprising recovering beta-alanine or beta-alanine-derived compound from the medium or microorganism according to the culturing.

9. A composition for producing beta-alanine or beta-alanine-derived compound, comprising a *Corynebacterium* sp. microorganism, into which aspartate 1-decarboxylase derived from *Tribolium castaneum* or a polynucleotide encoding the same is introduced.
